# EUROPÄISCHE PATENTSCHRIFT

(11) **EP 3 450 976 B1**
(45) Veröffentlichungstag und Bekanntmachung des Hinweises auf die Patenterteilung: **20.10.2021**
(21) Anmeldenummer: 18191132.2
(22) Anmeldetag: 28.08.2018
(51) Int. Cl.: G01N 33/18

(54) **VERFAHREN UND VORRICHTUNG ZUR BESTIMMUNG DES CHEMISCHEN SAUERSTOFFBEDARFS MIT THERMISCHEM PROBENAUFSCHLUSS**
METHOD AND DEVICE FOR DETERMINING CHEMICAL OXYGEN REQUIREMENTS WITH THERMAL SAMPLE DECOMPOSITION
PROCÉDÉ ET APPAREIL DE DÉTERMINATION DE LA DEMANDE CHIMIQUE EN OXYGÈNE PAR TRAITEMENT THERMIQUE D'ÉCHANTILLONS

(30) Priorität: 05.09.2017 DE 102017120386
(43) Veröffentlichungstag der Anmeldung: 06.03.2019
(73) Patentinhaber: LAR Process Analysers AG, 12057 Berlin (DE)
(72) Erfinder: Arts, Dr. Werner, 10825 Berlin (DE)
(74) Vertreter: Heinze, Ekkehard

(56) Entgegenhaltungen:
- DE-A1-102008 010 581
- DE-B3- 10 240 410
- Wolfgang Genthe ET AL: "Total Oxygen Demand (TOD) An Alternative Parameter For Real-Time Monitoring Of Wastewater Organics", , März 2015 (2015-03), XP055556847, Gefunden im Internet: URL:https://web.archive.org/web/2016123102 2549/https://www.wateronline.com/doc/total -oxygen-demand-tod-an-alternative-paramete r-for-real-time-monitoring-of-wastewater-o rganics-0001 [gefunden am 2019-02-14]
- Anonymous: "COD-Chemical Oxygen Demand - LAR Process Analysers", , 29. Juli 2016 (2016-07-29), XP055557029, Gefunden im Internet: URL:https://web.archive.org/web/2016072911 5612/https://www.lar.com/products/cod-anal ysis/cod-chemical-oxygen-demand.html [gefunden am 2019-02-14]
- VERNON A. STENGER ET AL: "Rapid method for determination of chemical oxygen demand", ANALYTICAL CHEMISTRY, Bd. 39, Nr. 2, Februar 1967 (1967-02), Seiten 206-211, XP055556445, US ISSN: 0003-2700, DOI: 10.1021/ac60246a003

## Beschreibung

Die Erfindung betrifft ein Verfahren zur Bestimmung des chemischen Sauerstoffbedarfs mit thermischem Probenaufschluss. Sie betrifft des Weiteren eine zur Durchführung dieses Verfahrens geeignete Vorrichtung.

Für einen umweltfreundlichen und effizienten Betrieb von Abwassernetzen, einschließlich der zugehörigen Kläranlagen, sind qualitative und quantitative Bestimmungen der wichtigsten das Abwasser belastenden Inhaltsstoffe unumgänglich. Mit der zunehmenden Erhöhung der Umweltstandards steigen auch die Anforderungen an die Genauigkeit, Reproduzierbarkeit und Vergleichbarkeit der entsprechenden Messwerte, auch wenn diese mit unterschiedlichen Messverfahren gewonnen wurden. Den bestehenden Anforderungen kann - auch mit Blick auf den Zeitbedarf für die Erfassung und Auswertung der Messwerte und die wünschenswerten Reaktionszeiten der Kläranlagen-Steuerung - am besten durch Erfassung sogenannter Summenparameter entsprochen werden.

Der Chemische Sauerstoffbedarf (CSB; englisch chemical oxygen demand, COD) ist als Summenparameter ein Maß für die Summe aller im Wasser vorhandenen, unter bestimmten Bedingungen oxidierbaren Stoffe. Er gibt die Menge an Sauerstoff (in mg/l) an, die zu ihrer Oxidation benötigt würde, wenn Sauerstoff das Oxidationsmittel wäre. Es werden auch die Bezeichnungen "Oxidierbarkeit Cr-VI" (Chromat-Verbrauch, wenn Chromat das Oxidationsmittel wäre) oder "Oxidierbarkeit Mn-VII" (Kaliumpermanganatverbrauch) verwendet. Der chemische Sauerstoffbedarf dient als Beurteilung für Schadstoffe, die ins Abwasser abgegeben oder die in einem Zeitraum entsorgt wurden.

Zur herkömmlichen Ermittlung des CSB wird eine Wasserprobe mit Schwefelsäure stark angesäuert und mit einer vorgegebenen genauen Menge des starken Oxidationsmittels Kaliumdichromat (K₂Cr₂O₇), unter Zusatz von Silbersulfat als Katalysator ,erhitzt. Bei chloridhaltigen Proben muss das Chlorid zuvor entfernt oder mit Quecksilbersulfat maskiert werden, damit eine Oxidation zu Chlor nicht den Messwert fälschlich erhöht. Die Menge an verbrauchtem Dichromat wird über Bestimmung des verbliebenen Dichromats berechnet und daraus die äquivalente Menge Sauerstoff O₂ bestimmt. Üblicherweise wird die verbliebene Menge des Dichromats titrimetrisch mit Ammoniumeisen(II)-Sulfat-Lösung und Ferroin-Indikator bestimmt (Verfahren DEV H41, H43 und H44, DIN 38409).

Das beschriebene Verfahren erfordert den Einsatz potentiell gefährlicher Chemikalien und ist zudem relativ zeitaufwändig, so dass es weder strenge Umweltschutz- und Arbeitsschutzbedingungen erfüllt noch ohne weiteres für den so genannten Online-Betrieb (Betrieb am Einlauf einer Kläranlage zu deren zeitnaher Steuerung) geeignet ist. Zudem ist das Verfahren nur bedingt automatisierbar.

Es wird daher seit Langem nach alternativen Verfahren zur CSB-Bestimmung gesucht, und solche wurden auch entwickelt und sind im praktischen Einsatz; vgl. etwa die DE 102 40410 B3 der Anmelderin. Diese beschreibt einen katalysatorfreien thermischen Aufschluss der zu untersuchenden Wasser- bzw. Abwasserprobe in einem Reaktionsgefäß. Dieses Verfahren lehnt sich an das amerikanische Standardverfahren zur Ermittlung des totalen Sauerstoffbedarfs TOD (ASTM D62 38-98) an. In dem das Reaktionsgefäß verlassende Verbrennungsgas wird der Sauerstoffverbrauch mittels eines O₂-Detektors (im Vergleich zum exakt vorbestimmten Sauerstoffgehalt des dem Reaktionsgefäß zugeführten Trägergases) bestimmt, und hieraus lässt sich ein CSB-Wert errechnen. Das letztgenannte Verfahren ist weit umweltfreundlicher und schneller als das herkömmliche Kaliumdichromat-Verfahren.

In Gegenwart von Salzen wie Sulfaten und Nitraten führt das von der Anmelderin entwickelte Verfahren jedoch zu signifikanten Unterbefunden. Hintergrund hierfür ist die Tatsache, dass im Verfahren der Oxidation durch Hochtemperaturaufschluss aus den genannten Störstoffen Sauerstoffbestandteile freigesetzt werden. Diese freigesetzten Sauerstoffbestandteile reduzieren die Sauerstoffzehrung durch die Oxidation in der Probe vorhandenen organischen Bestandteile. Je nach Konzentration der Störstoffe weichen somit die Ergebnisse des Verfahrens der Oxidation durch Hochtemperaturaufschluss zum Teil erheblich von den Ergebnissen des Kaliumdichromat-Verfahrens ab.

Der Erfindung liegt daher die Aufgabe zu Grunde, ein Verfahren und eine hierfür geeignete Vorrichtung anzugeben, die zu deutlich besser mit den Ergebnissen herkömmlicher Verfahren vergleichbaren CSB-Messwerten auch bei stark durch Sulfate und/oder Nitrate belasteten Proben führen.

Diese Aufgabe wird gemäß einem ersten Aspekt der Erfindung verfahrensseitig durch ein Verfahren mit den Merkmalen des Anspruchs 1 und vorrichtungsseitig durch eine Vorrichtung mit den Merkmalen des Anspruchs 6 gelöst. Zweckmäßige Fortbildungen des Erfindungsgedankens sind Gegenstand der abhängigen Ansprüche. Gemäß einem zweiten, relativ unabhängigen Aspekt der Erfindung wird die Aufgabe gelöst durch ein Verfahren mit den Merkmalen des Anspruchs 8 bzw. eine Vorrichtung mit den Merkmalen des Anspruchs 10.

Die Erfindung schließt, gemäß beiden Aspekten, den Gedanken ein, einen womöglich störenden Einfluss eines relativ hohen Gehaltes an Sulfaten und/oder Nitraten im Wasser oder Abwasser für den aufgrund einer Hochtemperaturoxidation ermittelten CSB-Wert unwirksam zu machen. Dieses Vorgehen haben die Erfinder sich aus dem Umstand abgeleitet, dass bei den bekannten Verfahren unter Bestimmung des Chromat-Verbrauchs oder des Kaliumpermanganat-Verbrauchs ein Sulfat- oder Nitrat-Gehalt prinzipbedingt keine Rolle spielt. Weiterhin gehört zur Erfindung der Gedanke, hierfür entweder die Sulfat- bzw. Nitrationen mittels eines Ionenaustauschs vor der CSB-Messung aus der Probe zu entfernen oder sie in der Probe zu belassen und ihren störenden Einfluss durch separate Messung/en und anschließende messwertabhängige Korrektur des initial ermittelten CSB-Wertes zu berücksichtigen.

Bei der Ausführungsvariante mit vorgeschaltetem Ionenaustausch kann vorteilhaft ein kommerziell verfügbarer Mischbettionentauscher eingesetzt werden, der neben den Sulfat- bzw. Nitrationen (Anionen) auch Kationen aus der Wasserprobe entfernt, wie z. B. Eisen (II)- oder Amoniumionen.

In einer Ausführungsform der Erfindung wird die Detektion des Sauerstoffgehaltes sowie des Sulfat- und Nitratgehaltes an ein und derselben Wasserprobe nach deren thermischer Oxidation ausgeführt. Es wird also das Abgas des Reaktionsgefäßes einerseits dem Sauerstoffdetektor und andererseits einem Detektor für den Sulfatgehalt und/oder einem Detektor für den Nitratgehalt zugeführt. Hierbei ist darauf zu achten, dass nicht durch Vorrichtungskomponenten, die zwischen dem Reaktionsgefäßausgang und dem jeweiligen Detektor angeordnet sind (etwa eine Säurefalle) eine Verfälschung der jeweiligen Detektionswerte erfolgen kann.

In einer alternativen Verfahrensdurchführung ist vorgesehen, dass die Wasserprobe in mindestens zwei Teil-Proben aufgeteilt wird und die Detektion des Sauerstoffgehaltes nach thermischer Oxidation der ersten Teilprobe ausgeführt wird und die Detektion des Sulfat- und/oder Nitratgehaltes an der zweiten und optional einer dritten Teil-Probe ausgeführt wird. Dies ist zeitaufwändiger, hat aber gegebenenfalls den Vorteil, dass die Bestimmung des Sulfat- und/oder Nitratgehaltes mit größerer Genauigkeit erfolgen kann. In einer Ausgestaltung ist vorgesehen, dass auch die zweite und optional dritte Teil-Probe zur Detektion des Sulfat- und/oder Nitratgehaltes einer thermischen Oxidation unterzogen wird/werden. Grundsätzlich kann der Sulfat- bzw. Nitratgehalt aber auch ohne einen solchen Schritt bestimmt werden.

Entsprechend der erfindungsgemäßen Verfahrensdurchführung werden zur Detektion des Sulfatgehaltes ein elektrochemischer SO₂-Gasdetektor und/oder zur Detektion des Nitratgehaltes ein elektrochemischer oder Chemolumineszenz-NOₓ-Detektor eingesetzt.

## Patentansprüche

1. Verfahren zur Bestimmung des chemischen Sauerstoffbedarfs, CSB, einer Wasserprobe durch thermische Oxydation der Wasserprobe in einem geschlossenen Reaktionsgefäß in einem Trägergas mit vorbestimmtem Sauerstoffgehalt und anschließende Detektion des Sauerstoffgehaltes im Abgas des Reaktionsgefäßes und Berechnung des CSB-Wertes aus dem detektierten Sauerstoffgehalt,
**dadurch gekennzeichnet, dass**
der Gehalt an Sulfaten und/oder Nitraten in der Wasserprobe durch jeweils einen dedizierten Detektor erfasst und eine rechnerische Korrektur des aufgrund des Sauerstoffgehaltes berechneten CSB-Wertes mittels eines vorbestimmten Korrekturalgorithmus aufgrund des detektierten Sulfat- und/oder Nitratgehaltswertes ausgeführt wird, wobei zur Detektion des Sulfatgehaltes ein elektrochemischer SO₂-Gasdetektor und/oder zur Detektion des Nitratgehaltes ein elektrochemischer oder Chemolumineszenz-NOₓ-Detektor eingesetzt wird.

2. Verfahren nach Anspruch 1, wobei die Detektion des Sauerstoffgehaltes sowie des Sulfat- und/oder Nitratgehaltes an ein und derselben Wasserprobe nach deren thermischer Oxidation ausgeführt wird.

3. Verfahren nach Anspruch 1, wobei die Wasserprobe in mindestens zwei Teil-Proben aufgeteilt wird und die Detektion des Sauerstoffgehaltes nach thermischer Oxidation der ersten Teilprobe ausgeführt wird und die Detektion des Sulfat- und/oder Nitratgehaltes an der zweiten und optional einer dritten Teil-Probe ausgeführt wird.

4. Verfahren nach Anspruch 3, wobei auch die zweite und optional dritte Teil-Probe zur Detektion des Sulfat- und/oder Nitratgehaltes einer thermischen Oxidation unterzogen wird/werden.

5. Vorrichtung zur Durchführung des Verfahrens nach einem der vorangehenden Ansprüche, welche aufweist:
- ein Reaktionsgefäß zur thermischen Oxidation einer Wasserprobe, mit einer Beschickungsöffnung zur Zufuhr der Wasserprobe,
- einen Gaseinlass zur Zuführung von Trägergas und einem Gasauslass zur Abführung des Abgases der thermischen Oxidation
- eine Trägergaserzeugungseinrichtung zur Erzeugung eines Trägergases mit vorbestimmtem Sauerstoffgehalt und Zuführung des Trägergases zu einem Einlass des Reaktionsgefäßes,
- einer Abgasausleitungseinrichtung zur Ausleitung des bei der thermischen Oxidation entstehenden Abgases,
- einen im Abgasstrom angeordneten Sauerstoffdetektor zur Bestimmung des Sauerstoffgehaltes im Abgas,
- eine CSB-Berechnungsstufe zur Berechnung des CSB-Wertes der Wasserprobe aufgrund des Ausgangssignals des Sauerstoffdetektors,
**gekennzeichnet durch**
- einen elektrochemischen SO₂-Detektor für den Sulfatgehalt der Wasserprobe und/oder
- einen elektrochemischen oder Chemilumineszenz-NOₓ-Detektor für den Nitratgehalt der Wasserprobe und
- eine Kompensations-Berechnungsstufe zur rechnerischen Korrektur des in der CSB-Berechnungsstufe ermittelten CSB-Wertes aufgrund des Ausgangssignals des Detektors für den Sulfatgehalt und/oder des Detektors für den Nitratgehalt.

6. Verfahren zur Bestimmung des chemischen Sauerstoffbedarfs, CSB, einer Wasserprobe durch thermische Oxidation der Wasserprobe in einem geschlossenen Reaktionsgefäß in einem Trägergas mit vorbestimmtem Sauerstoffgehalt und anschließende Detektion des Sauerstoffgehaltes im Abgas des Reaktionsgefäßes und Berechnung des CSB aus dem detektierten Sauerstoffgehalt,
**dadurch gekennzeichnet, dass**
die Wasserprobe vor der thermischen Oxidation einer Vorbehandlung mittels eines Ionentauschers zur Entfernung von Sulfat- und/oder Nitrationen unterzogen wird.

7. Verfahren nach Anspruch 6, wobei die Vorbehandlung mittels eines Ionentauschers unter Einsatz einer Mischbett-Ionentauschereinrichtung zur gleichzeitigen Entfernung von Anionen und Kationen aus der Wasserprobe ausgeführt wird.

8. Vorrichtung zur Durchführung des Verfahrens nach Anspruch 6 oder 7, welche aufweist:
- ein Reaktionsgefäß zur thermischen Oxidation einer Wasserprobe, mit einer Beschickungsöffnung zur Zufuhr der Wasserprobe,
- einen Gaseinlass zur Zuführung von Trägergas und einem Gasauslass zur Abführung des Abgases der thermischen Oxidation
- eine Trägergaserzeugungseinrichtung zur Erzeugung eines Trägergases mit vorbestimmtem Sauerstoffgehalt und Zuführung des Trägergases zu einem Einlass des Reaktionsgefäßes,
- einer Abgasausleitungseinrichtung zur Ausleitung des bei der thermischen Oxidation entstehenden Abgases,
- einen im Abgasstrom angeordneten Sauerstoffdetektor zur Bestimmung des Sauerstoffgehaltes im Abgas,
- eine CSB-Berechnungsstufe zur Berechnung des CSB-Wertes der Wasserprobe aufgrund des Ausgangssignals des Sauerstoffdetektors,
**gekennzeichnet durch**
- eine Einrichtung zur Bewirkung eines Ionenaustauschs in der Wasserprobe zur Entfernung der Sulfat- und/oder Nitrationen aus der Wasserprobe.

9. Vorrichtung nach Anspruch 8, wobei die Einrichtung zur Bewirkung eines Ionenaustauschs eine Mischbett-Ionentauschereinrichtung aufweist.

10. Vorrichtung nach Anspruch 8, wobei die Einrichtung zur Bewirkung eines Ionenaustauschs eine Mischbett-Ionentauschereinrichtung eine Ionenaustauschersäule aufweist.

## Claims

1. A method for determining chemical oxygen requirements (CSB) of a water sample by thermal oxidation of the water sample in a closed reaction vessel in a carrier gas having a predetermined oxygen content, and subsequent detection of the oxygen content in the exhaust gas of the reaction vessel, and calculation of the CSB value from the detected oxygen content,
**characterized in that**
the content of sulfates and/or nitrates in the water sample is in each case acquired by a dedicated detector, and an arithmetical correction of the CSB value calculated based on the oxygen content is performed by means of a predetermined correction algorithm of the detected sulfate content value and/or nitrate content value, wherein an electrochemical SO₂ gas detector is used for detecting the sulfate content and/or an electrochemical or chemiluminescence NOₓ detector is used for detecting the nitrate content.

2. The method according to claim 1, wherein the detection of the oxygen content as well as of the sulfate content and/or nitrate content is performed in one and the same water sample after its thermal oxidation.

3. The method according to claim 1, wherein the water sample is divided in at least two sub-samples, and the detection of the oxygen content is performed after the thermal oxidation of the first sub-sample, and the detection of the sulfate content and/or nitrate content is performed on the second sub-sample and optionally on a third sub-sample.

4. The method according to claim 3, wherein the second and optionally third sub-sample is/are subjected to a thermal oxidation for detecting the sulfate and/or nitrate contents.

5. A device for implementing the method according to any one of the preceding claims, including:
- a reaction vessel for thermal oxidation of a water sample, having a feeding opening for feeding the water sample,
- a gas inlet for feeding a carrier gas, and a gas outlet for discharging the exhaust gas of the thermal oxidation,
- a carrier gas generation device for generating the carrier gas having a predetermined oxygen content, and for feeding the carrier gas to an inlet of the reaction vessel,
- a exhaust gas discharging device for discharging the exhaust gas developing during the thermal oxidation,
- an oxygen detector arranged within the exhaust gas flow for determining the oxygen content within the exhaust gas,
- a CSB calculation stage for calculating the CSB value of the water sample based on the output signal of the oxygen detector,
**characterized by**
- an electrochemical SO₂ detector for the sulfate content of the water sample and/or
- an electrochemical or chemiluminescence NOₓ detector for the nitrate content of the water sample, and
- a compensation calculation stage for arithmetically correcting the CSB value determined in the CSB calculation stage based on the output signal of the detector for the sulfate content and/or the detector for the nitrate content.

6. A method for determining chemical oxygen requirements (CSB) of a water sample by thermal oxidation of the water sample in a closed reaction vessel in a carrier gas having a predetermined oxygen content, and subsequent detection of the oxygen content in the exhaust gas of the reaction vessel, and calculation of the CSB from the detected oxygen content,
**characterized in that**
prior to the thermal oxidation, the water sample is subjected to a pre-treatment by means of an ion exchanger for removing sulfate ions and/or nitrate ions.

7. The method according to claim 6, wherein the pre-treatment is performed by means of an ion exchanger using a mixed bed ion exchanger apparatus for simultaneously removing anions and cations from the water sample.

8. A device for implementing the method according to claim 6 or 7, including:
- a reaction vessel for thermal oxidation of a water sample, having a feeding opening for feeding the water sample,
- a gas inlet for feeding a carrier gas, and a gas outlet for discharging the exhaust gas of the thermal oxidation,
- a carrier gas generation device for generating the carrier gas having a predetermined oxygen content, and for feeding the carrier gas to an inlet of the reaction vessel,
- a exhaust gas discharging device for discharging the exhaust gas developing during the thermal oxidation,
- an oxygen detector arranged within the exhaust gas flow for determining the oxygen content within the exhaust gas,
- a CSB calculation stage for calculating the CSB value of the water sample based on the output signal of the oxygen detector,
**characterized by**
- an apparatus for effecting an ion exchange within the water sample for removing the sulfate ions and/or nitrate ions from the water sample.

9. The device according to claim 8, wherein the apparatus for effecting an ion exchange includes a mixed bed ion exchanger apparatus.

10. The device according to claim 8, wherein the apparatus for effecting an ion exchange includes a mixed bed ion exchanger apparatus having a ion exchanger column.

## Revendications

1. Procédé pour déterminer la demande chimique en oxygène, DCO, d'un échantillon d'eau par oxydation thermique de l'échantillon d'eau dans un récipient de réaction fermé dans un gaz porteur ayant une teneur en oxygène prédéterminée, puis par détection de la teneur en oxygène dans les effluents gazeux du récipient de réaction et par calcul de la valeur DCO à partir de la teneur en oxygène détectée,
**caractérisé en ce que**
la teneur en sulfates et/ou en nitrates de l'échantillon d'eau est détectée par un détecteur dédié respectif, et une correction par calcul de la valeur DCO calculée sur la base de la teneur en oxygène est effectuée au moyen d'un algorithme de correction prédéterminé sur la base de la valeur détectée de la teneur en sulfates et/ou en nitrates, et
un détecteur de gaz SO₂ électrochimique est utilisé pour la détection de la teneur en sulfates, et/ou un détecteur de NOₓ électrochimique ou à chimioluminescence est utilisé pour la détection de la teneur en nitrates.

2. Procédé selon la revendication 1, dans lequel la détection de la teneur en oxygène et de la teneur en sulfates et/ou en nitrates est effectuée sur un seul et même échantillon d'eau après son oxydation thermique.

3. Procédé selon la revendication 1, dans lequel l'échantillon d'eau est subdivisé en au moins deux échantillons partiels, et la détection de la teneur en oxygène est effectuée après oxydation thermique du premier échantillon partiel, et la détection de la teneur en sulfates et/ou en nitrates est effectuée sur le deuxième échantillon partiel et optionnellement sur un troisième échantillon partiel.

4. Procédé selon la revendication 3, dans lequel le deuxième et optionnellement le troisième échantillon partiel est/sont également soumis à une oxydation thermique pour la détection de la teneur en sulfates et/ou en nitrates.

5. Dispositif pour la mise en œuvre du procédé selon l'une des revendications précédentes, comprenant :
- un récipient de réaction pour l'oxydation thermique d'un échantillon d'eau, présentant un orifice d'alimentation pour alimenter l'échantillon d'eau,
- une entrée de gaz pour fournir un gaz porteur, et une sortie de gaz pour évacuer les effluents gazeux de l'oxydation thermique,
- un moyen de génération de gaz porteur pour générer un gaz porteur ayant une teneur en oxygène prédéterminée et pour fournir le gaz porteur à une entrée du récipient de réaction,
- un moyen d'évacuation des effluents gazeux pour évacuer les effluents gazeux produits pendant l'oxydation thermique,
- un détecteur d'oxygène disposé dans le flux des effluents gazeux pour déterminer la teneur en oxygène dans les effluents gazeux,
- un étage de calcul de DCO pour calculer la valeur DCO de l'échantillon d'eau sur la base du signal de sortie du détecteur d'oxygène,
**caractérisé par**
- un détecteur de SO₂ électrochimique de la teneur en sulfates de l'échantillon d'eau, et/ou
- un détecteur de NOₓ électrochimique ou à chimioluminescence de la teneur en nitrates de l'échantillon d'eau, et
- un étage de calcul de compensation pour la correction par calcul de la valeur DCO déterminée dans l'étape de calcul de DCO sur la base du signal de sortie du détecteur de la teneur en sulfates et/ou du détecteur de la teneur en nitrates.

6. Procédé pour déterminer la demande chimique en oxygène, DCO, d'un échantillon d'eau par oxydation thermique de l'échantillon d'eau dans un récipient de réaction fermé dans un gaz porteur ayant une teneur en oxygène prédéterminée, puis par détection de la teneur en oxygène dans les effluents gazeux du récipient de réaction et par calcul de la DCO à partir de la teneur en oxygène détectée,
**caractérisé en ce que**
avant l'oxydation thermique, l'échantillon d'eau est soumis à un prétraitement au moyen d'un échangeur d'ions pour éliminer les ions sulfate et/ou nitrate.

7. Procédé selon la revendication 6, dans lequel le prétraitement au moyen d'un échangeur d'ions est réalisé en utilisant un moyen d'échange d'ions à lit mixte pour l'élimination simultanée des anions et des cations hors de l'échantillon d'eau.

8. Dispositif pour la mise en oeuvre du procédé selon la revendication 6 ou 7, comprenant :
- un récipient de réaction pour l'oxydation thermique d'un échantillon d'eau, présentant un orifice d'alimentation pour alimenter l'échantillon d'eau,
- une entrée de gaz pour fournir un gaz porteur, et une sortie de gaz pour évacuer les effluents gazeux de l'oxydation thermique,
- un moyen de génération de gaz porteur pour générer un gaz porteur ayant une teneur en oxygène prédéterminée et pour fournir le gaz porteur à une entrée du récipient de réaction,
- un moyen d'évacuation d'effluents gazeux pour évacuer les effluents gazeux produits pendant l'oxydation thermique,
- un détecteur d'oxygène disposé dans le flux des effluents gazeux pour déterminer la teneur en oxygène dans les effluents gazeux,
- un étage de calcul de DCO pour calculer la valeur DCO de l'échantillon d'eau sur la base du signal de sortie du détecteur d'oxygène,
**caractérisé par**
- un moyen pour entraîner un échange d'ions dans l'échantillon d'eau afin d'éliminer les ions sulfate et/ou nitrate hors de l'échantillon d'eau.

9. Dispositif selon la revendication 8,
dans lequel le moyen pour entraîner un échange d'ions comprend un moyen d'échange d'ions à lit mixte.

10. Dispositif selon la revendication 8,
dans lequel le moyen pour entraîner l'échange d'ions comprend un moyen d'échange d'ions à lit mixte et une colonne d'échange d'ions.
